# EUROPÄISCHE PATENTSCHRIFT

(11) **EP 2 302 610 B1**
(45) Veröffentlichungstag und Bekanntmachung des Hinweises auf die Patenterteilung: **17.12.2014**
(21) Anmeldenummer: 10173745.0
(22) Anmeldetag: 23.08.2010
(51) Int. Cl.: G09F 3/02, A61M 5/32, A61M 5/31

(54) **Etikett zum Anbringen an einem Spritzenkörper und Spritzenkörper**
Label for attaching to a syringe body and syringe body
Etiquette destinée à l'application sur un corps de seringue et corps de seringue

(30) Priorität: 01.09.2009 DE 102009039572
(43) Veröffentlichungstag der Anmeldung: 30.03.2011
(73) Patentinhaber: Schreiner Group GmbH & Co. KG, 85764 Oberschleissheim (DE)
(72) Erfinder: Moosheimer, Ulrich, 85411, Hohenkammer (DE); Hölzl, Timo, 89284, Pfaffenhofen (DE)
(74) Vertreter: Epping - Hermann - Fischer

(56) Entgegenhaltungen:
- WO-A1-2006/105807
- DE-U1-202008 017 330

## Beschreibung

Die Anmeldung betrifft ein Etikett zum Anbringen an einen Spritzenkörper sowie einen mit diesem Etikett versehenen Spritzenkörper.

In der Medizin und anderen Anwendungsgebieten werden Wirkstoffe, beispielsweise Medikamente mit Hilfe einer Spritze verabreicht, wobei die Spritze einen Spritzenkörper (meist aus Kunststoff oder Glas) und einen in ihr verschiebbaren Stopfen bzw. Kolben umfasst. Die Spritze ist an ihrem vorderen Ende mit einer Spritzennadel verbindbar oder bereits verbunden. Fertigungstechnisch werden Spritzenkörper und Spritzennadel oft einteilig gefertigt, beispielsweise in Form einer in einem gläsernen Spritzenkörper eingelassenen Spritzennadel.

Um das Verletzungsrisiko bei der Handhabung der Spritze und bei ihrem Transport zu verringern, wird die Spritzennadel mit einer Kappe abgedeckt, die auf die Spritzennadel aufgesetzt wird. Dabei wird die Kappe in axialer Richtung gegen die Einfassung der Spritzennadel gedrückt.

Bei der Spritzenherstellung und ihrer Konfektionierung geschieht dies maschinell. Nach Gebrauch der Spritze jedoch besteht ein Verletzungsrisiko, wenn die Kappe auf diese Weise wieder auf die Nadel bzw. das sie umgebende vordere Ende des Spritzenkörpers aufgedrückt wird.

Deshalb ist es im Bereich beispielsweise der Medizin üblich, Spritzen zusätzlich mit einer Nadelaufnahmeeinrichtung zu versehen, die seitlich neben der Einfassung der Spritzennadel am Spritzenkörper montiert ist. Eine solche Nadelaufnahmeeinrichtung ist seitlich gegen die Spritzennadel andrückbar, wobei die Spritzennadel ihrer Länge nach in eine entsprechende Ausnehmung oder Vertiefung der Nadelaufnahmeeinrichtung eingelassen wird. Nach Gebrauch einer Spritze, insbesondere einer Medikamentenspritze soll so die durch die offenliegende Spritzennadel bedingte Verletzungsgefahr herabgesetzt werden, und zwar ohne dass hierzu die ursprünglich aufsitzende Kappe wieder aufgesetzt werden müsste.

Nadelaufnahmeeinrichtungen der hier oben beschriebenen Art sind aus US 3,658,061 und aus US 2007/0260191 A1 bekannt.

Aus WO 2006/105807 A1 ist bekannt, eine solche Nadelaufnahmeeinrichtung zum späteren Schutz der Spritzennadel in Form eines Etiketts auszubilden. Das Etikett wird unter Verwendung von Folien hergestellt, wobei ein erster Folienteil um den Spritzenkörper umwickelbar ist. Die gegen die Spritzennadel andrückbare Nadelaufnahmeeinrichtung ist mit Hilfe eines Übergangsbereichs schwenkbar mit dem ersten Folienteil verbunden. Während der erste Folienteil des Etiketts den Spritzenkörper umgibt und auf dessen Außenwandung aufgeklebt ist, ragen der Übergangsbereich, zumindest aber die Nadelaufnahmeeinrichtung zur Sicherung der Spritzennadel in einer ersten, axialen Richtung über die Schulter des Spritzenkörpers hinaus. Nach dem Verabreichen der Spritzenflüssigkeit, beispielsweise der Medikamentenlösung wird die Nadelaufnahmeeinrichtung seitlich an die offenliegende Spritzennadel angedrückt.

Ein weiteres aus dem Stand der Technik bekanntes Etikett ist offenbart in DE 20 2008 017 330.

Problematisch ist hierbei die Ausbildung des Übergangsbereichs zwischen dem aufgeklebten ersten Folienteil und der Nadelaufnahmeeinrichtung für die Spritzennadel. Dieser Übergangsbereich dient zur beweglichen, insbesondere schwenkbaren Befestigung der Nadelaufnahmeeinrichtung an dem Spritzenkörper. Je nach Winkelstellung der Nadelaufnahmeeinrichtung relativ zur Spritzennadel ist die Nadelaufnahmeeinrichtung beim Verabreichen der Spritze mehr oder weniger im Wege. Es gestaltet sich schwierig, den Übergangsbereich so auszubilden, dass definierte Winkelstellungen der vorstehenden Nadelaufnahmeeinrichtung relativ zur Spritzennadel sich von selbst einstellen, ohne dass der Anwender die Nadelaufnahmeeinrichtung zurückschwenken oder die Winkelstellung von Hand nachkorrigieren müsste. Insbesondere dann, wenn beim Auspacken oder aus sonstigen Gründen die Nadelaufnahmeeinrichtung bereits mehrfach vor- und zurückgeschwenkt wurde, wird bei herkömmlichen Ausgestaltungen nicht zuverlässig erreicht, dass die Nadelaufnahmeeinrichtung stets erneut die vordefinierten Winkelstellungen, beispielsweise nahe an der Spritzennadel und in einem großen Winkel verdreht zu ihr wieder einnimmt. Statt dessen wird der Übergangsbereich durch mehrfaches Hin- und Herschwenken geschwächt oder gar beschädigt.

Es wäre daher erstrebenswert, ein Etikett zum Anbringen an einen Spritzenkörper bereitzustellen, bei dem sich die Orientierung oder Winkelposition der Nadelaufnahmeeinrichtung relativ zur Richtung der Spritzennadel zuverlässig wieder von selbst einstellt, insbesondere auch bei mehrmaligem Hin- und Herschwenken der Nadelaufnahmeeinrichtung.

Es wird ein Etikett zum Anbringen an einen Spritzenkörper vorgeschlagen, das Folgendes aufweist:
- einen ersten Folienteil, der um einen Spritzenkörper umwickelbar ist,
- eine Nadelaufnahmeeinrichtung für eine Spritzennadel zum Schutz vor Verletzungen durch die Spritzennadel, wobei die Nadelaufnahmeeinrichtung zumindest einen zweiten Folienteil und ein Kunststoffformteil, das gegen die Spritzennadel seitlich andrückbar ist, aufweist, und
- einen Übergangsbereich, durch den die Nadelaufnahmeeinrichtung schwenkbar mit dem ersten Folienteil verbunden ist, wobei der Übergangsbereich in einer ersten Richtung seitlich über den ersten Folienteil hinausragt und wobei das Etikett in dem Übergangsbereich eine oder mehrere Folien aufweist,
wobei im Übergangsbereich des Etiketts in mindestens einer Folie zumindest ein erstes Schwächungselement vorgesehen ist,
wobei das zumindest eine erste Schwächungselement eine von der ersten Richtung abweichende Orientierung besitzt.

Einige Ausführungsbeispiele werden nachstehend mit Bezug auf die Figuren beschrieben. Es zeigen:
- Figur 1: eine schematische Ansicht eines Ausführungsbeispiels eines Etiketts sowie einer damit zu versehenden Spritze,
- Figur 2: eine Querschnittsansicht zu Figur 1 mit aufgespendetem Etikett und zwei verschiedenen Winkelstellungen einer Nadelaufnahmeeinrichtung des Etiketts,
- Figur 3A: eine Querschnittsansicht eines Ausführungsbeispiels eines Etiketts entlang der Symmetrielinie der Nadelaufnahmeeinrichtung,
- Figur 3B: eine vergrößerte beispielhafte Detailansicht aus Figur 3A,
- Figur 4: eine vergrößerte Detailansicht des Etiketts aus Figur 1 gemäß einem ersten Ausführungsbeispiel,
- Figur 5: eine vergrößerte Detailansicht des Etiketts aus Figur 1 gemäß einem zweiten Ausführungsbeispiel,
- Figur 6: eine vergrößerte Detailansicht des Etiketts aus Figur 1 gemäß einem dritten Ausführungsbeispiel,
- Figur 7: eine vergrößerte Detailansicht des Etiketts aus Figur 1 gemäß einem vierten Ausführungsbeispiel,
- Figur 8: eine vergrößerte Detailansicht des Etiketts aus Figur 1 gemäß einem fünften Ausführungsbeispiel,
- Figur 9: eine vergrößerte Detailansicht des Etiketts aus Figur 1 gemäß einem sechsten Ausführungsbeispiel,
- Figur 10: eine alternative Ausführungsform mit einer Mikrostruktur der vorgesehenen Schwächungselemente, erläutert anhand des Ausführungsbeispiels aus Figur 5 und
- Figur 11: die Ausführungsform aus Figur 10 mit zusätzlicher Kennzeichnung der jeweiligen, durch die Mikrostruktur strukturierten Schwächungselemente.

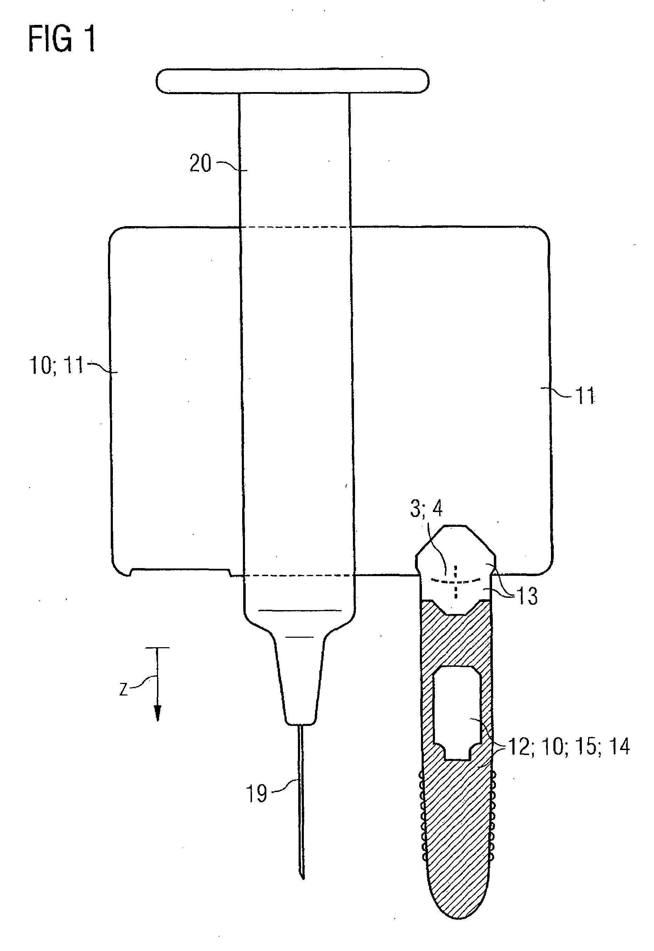

Figur 1 zeigt schematisch dargestellt eine Ausführungsform des vorgeschlagenen Etiketts 10 sowie eine damit zu umklebende Spritze 20, auf deren Spritzenkörper 20 das Etikett 10 aufgespendet wird. Dadurch umrollt der erste Folienteil 11, der in Figur 1 näherungsweise rechteckig ausgebildet ist, den Umfang des Spritzenkörpers 20 zumindest teilweise. Ein zweiter Folienteil 12 des Etiketts 10 hingegen ragt über den ersten Folienteil 11 seitlich in einer ersten Richtung, die der axialen Richtung z der Spritze entspricht, hinaus. Die axiale Richtung z ist diejenige Richtung, in welche die Spritzennadel 19 weist, die mit dem Spritzenkörper 20 verbunden oder verbindbar ist.

An dem zweiten Folienteil 12 des Etiketts 10 ist ein Kunststoffformteil 14 montiert, welches nach innen gegen die Spritzennadel 19 gedrückt werden kann. Dabei wird die Spritzennadel 19 umschlossen und kann so in eine entsprechende Einrastvorrichtung, beispielsweise in mehrere Haken bzw. Widerhaken des Kunststoffformteils 14 einrasten. Dadurch besteht nach Verabreichung der Spritzenflüssigkeit keine Verletzungsgefahr mehr, sobald die Spritzennadel 19 auf diese Weise gesichert wird.

Zwischen dem ersten Folienteil 11 und der Nadelaufnahmeeinrichtung 15 des Etiketts 10 ist ein Übergangsbereich 13 des Etiketts (zur flexiblen, d. h. biegsamen und somit schwenkbaren Verbindung der Nadelaufnahmeeinrichtung 15 samt dem aufgespendeten ersten Folienteil 11) vorgesehen. Der Übergangsbereich 13 sorgt nicht nur für eine schwenkbare und zugleich sichere Befestigung der Nadelaufnahmeeinrichtung 15 an dem Spritzenkörper 20, sondern auch dafür, dass sich die Orientierung der Nadelaufnahmeeinrichtung 15 in einer oder mehreren vordefinierten Winkelstellungen gegenüber der axialen Richtung z automatisch einstellt, damit die Aufnahmeeinrichtung 15 einerseits bei der Injektion nicht im Wege ist, andererseits jedoch anschließend ohne Mühe gegen die Spritzennadel 19 gedrückt werden kann.

Die Aufnahmeeinrichtung 15 umfasst somit außer dem Kunststoffformteil 14 auch noch einen zweiten Folienteil 12, der aus einer oder mehreren Folien gebildet sein kann. Der zweite Folienteil ist somit ein Flächenbereich der das Etikett im Wesentlichen ausmachenden Gesamtfolie, und zwar derjenige Folienteil, an den das Kunststoffformteil 14 angeklebt ist. Auch im ersten Folienteil 11 und/oder im Übergangsbereich 13 können eine oder mehrere Folien aufeinander vorgesehen sein, die dort zu einer Gesamtfolie verbunden sind.

Vorzugsweise ist eine erste Folie vorgesehen, deren Grundfläche sich über den ersten Folienteil 11, den Übergangsbereich 13 und die Nadelaufnahmeeinrichtung 15 erstreckt. Weiterhin ist vorzugsweise eine zweite Folie vorgesehen, die sich jedoch vorzugsweise nur über den Übergangsbereich 13 und die Nadelaufnahmeeinrichtung 15 erstreckt. Der erste Folienteil 11 ist somit vorzugsweise nur aus einer einzigen Folienschicht gebildet. Die übrigen Flächenbereiche sind vorzugsweise mehrschichtig (nämlich aus zumindest der ersten und der zweiten Folie) ausgebildet, insbesondere um das automatische Einstellen bestimmter Winkelstellungen, in welche die Nadelaufnahmeeinrichtung 15 bei und/oder nach der Injektion zeigen soll, zu erreichen.

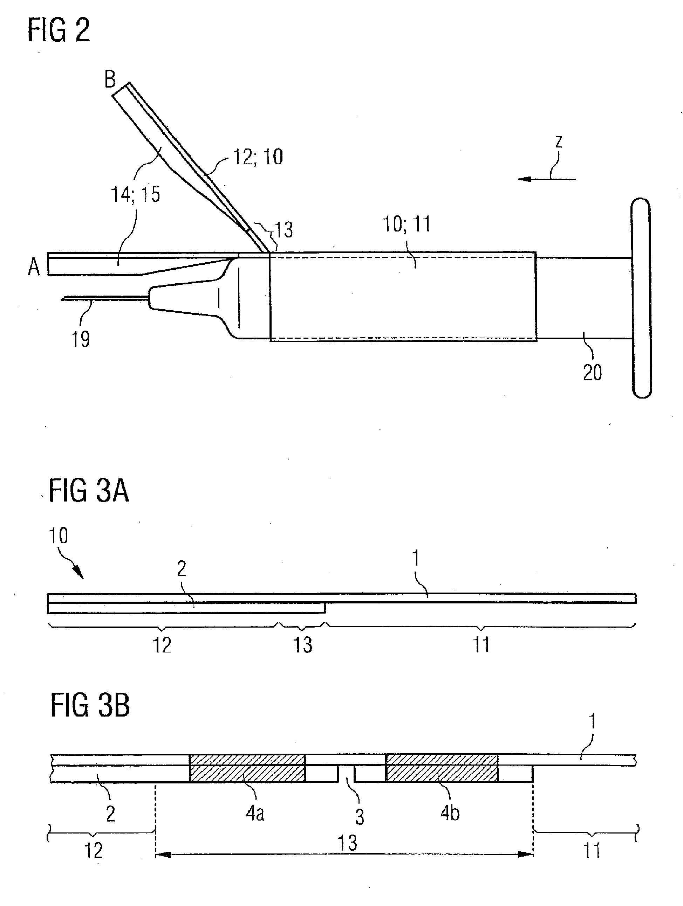

Figur 2 zeigt eine schematische Querschnittsansicht zu Figur 1, bei der das in Figur 1 noch lose Etikett 10 mit seinem ersten Folienteil 11 um den Spritzenkörper 20 gewickelt ist. Die Nadelaufnahmeeinrichtung 15 mit ihrem Kunststoffformteil 14 und dem zweiten Folienteil 12 ist in Figur 2 doppelt, nämlich in zwei verschiedene Winkelstellungen A und B weisend dargestellt. Während dieser Nadelschutz nach der Injektion nach unten, d.h. gegen die Spritzennadel 19 gedrückt wird, ist er vorher um maximal 180° zurückklappbar. Im Auslieferungszustand wird diese Nadelaufnahmeeinrichtung 15 in die Richtung z weisen, die der ersten Winkelstellung A von vorzugsweise 0° oder zumindest von zwischen 0° und 20° gegenüber der Richtung z entspricht.

Andererseits soll die Nadelaufnahmeeinrichtung 15 bei entsprechender Anfangsauslenkung von Hand anschließend von alleine möglichst eine zweite Winkelstellung B einnehmen, die vorzugsweise im Bereich zwischen 50° und 80° gegenüber der Richtung z liegt. Zwischen diesen beiden Winkelstellungen A, B sollte ein instabiler Winkelstellungsbereich liegen, in dem der Nadelschutz entweder von alleine wieder in Richtung der Winkelstellung A oder in Richtung der Winkelstellung B zurückschnellt. Die Winkelstellung B ermöglicht das problemlose Injizieren, wohingegen die Winkelstellung A nach der Injektion zum Andrücken gegen die Spritzennadel 19 vorteilhaft ist. Die Aufgabe des Übergangsbereichs 13, der beispielsweise aus zwei Folienschichten besteht, ist es, das spontane Einstellen der jeweiligen Winkelstellungen A oder B zu bewirken. Beispielsweise sollte bei einer größeren Auslenkung von mehr als 90° gegenüber der Richtung z die Nadelaufnahmeeinrichtung 15 wieder vorwärts in Richtung der Winkelstellung B vorschnellen und dort verbleiben, solange sie nicht durch den Anwender von Hand weiterbewegt wird.

Figur 3A zeigt eine Querschnittsansicht eines Ausführungsbeispiels eines Etiketts entlang der Symmetrielinie der Nadelaufnahmeeinrichtung, beispielsweise für das Etikett aus Figur 1 und 2. Dargestellt ist hier der Schichtenaufbau des Etiketts, welches ohne das Kunststoffformteil 14 abgebildet ist.

Wie aus Figur 3A ersichtlich ist, weist das Etikett eine erste Folie 1 und eine zweite Folie 2 auf, wobei die Grundfläche der ersten Folie 1 den ersten Folienteil 11, den Übergangsbereich 13 und den an das Kunststoffformteil 14 anzuklebenden zweite Folienteil 12 umfasst. Die Grundfläche der zweiten Folie 2 hingegen umfasst lediglich den Übergangsbereich 13 und den zweiten Folienteil 12. Vorzugsweise besteht die Etikettenfolie somit aus zwei Folien 1, 2. Vorzugsweise ist die zweite, untere Folie 2 dicker und/oder verwindungssteifer als die obere, erste Folie 1 ausgebildet.

Figur 3B zeigt eine vergrößerte Detailansicht aus Figur 3A, in der ebenfalls die Anordnung der Schwächungselemente 3, 4a, 4b angedeutet ist. Die in Figur 3B dargestellte Schnittebene verläuft wie in Figur 3A durch die Mitte der Nadelaufnahmeeinrichtung 15 (siehe die Draufsicht auf Figur 1). Somit verlaufen in Figur 3B die Schwächungs- oder Schnittlinien, die den Schwächungselementen 4a, 4b entsprechen, in der Zeichenebene; sie sind durch die schraffierten Gebiete dargestellt. Wie anhand Figur 3B erkennbar ist, sind bei diesem Ausführungsbeispiel die Schwächungselemente 4a, 4b sowohl in der oberen Folie 1 als auch in der unteren Folie 2 ausgebildet. Das erste Schwächungselement 3 hingegen ist nur in der unteren Folie ausgebildet, die entsprechend dem quer zur Zeichenebene verlaufenden Schnitt in der Mitte des Übergangsgebietes durchbrochen bzw. durchschnitten ist. Die genaue Anordnung der Schwächungs- bzw. Schnittlinien kann beispielsweise auch gemäß einem der nachstehend näher erläuterten Ausführungsbeispiele gewählt werden. Ebenso können diese und die nachstehenden Ausführungsbeispiele hinsichtlich der jeweiligen Folie oder Folien, in denen einzeln der jeweiligen Schwächungselemente auszubilden sind, variiert werden.

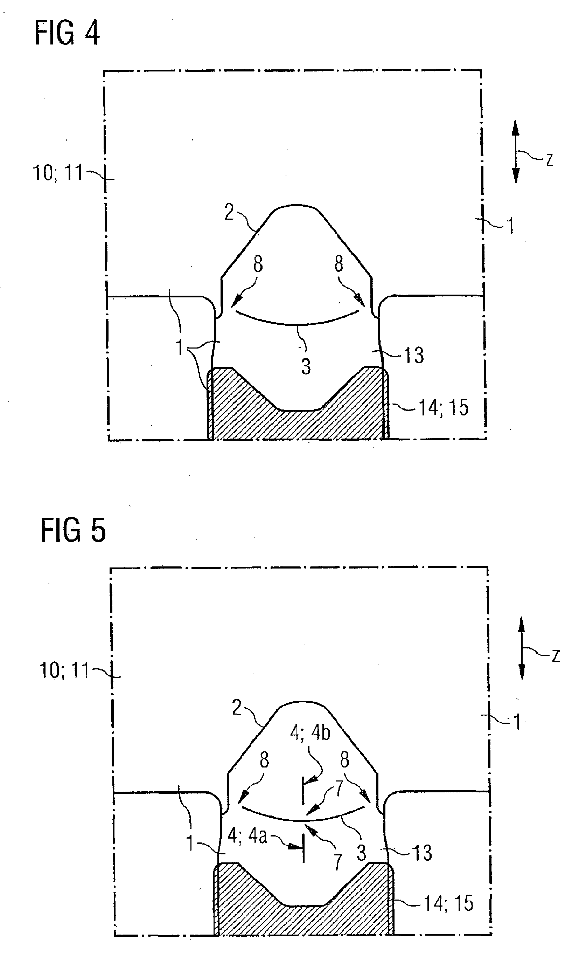

Figur 4 zeigt eine vergrößerte Detailansicht des Etiketts aus Figur 1 gemäß einem ersten Ausführungsbeispiel. Dargestellt ist der Übergangsbereich 13, der den ersten Folienteil 11 des Etiketts 10 mit der Nadelaufnahmeeinrichtung 15 des Etiketts verbindet. Deren Kunststoffformteil 14 ist in Figur 4 schraffiert dargestellt; das Formteil ist an der Unterseite des zweiten Folienteils 12, die der Spritzennadel zugewandt ist, angebracht.

Der Übergangsbereich 13 des Etiketts ist im wesentlichen aus einer oder mehreren Folien, beispielsweise Kunststofffolien und/oder Papierfolien gebildet. Die Folien des Übergangsbereichs 13 können unmittelbar in die Folien des ersten und/oder des zweiten Folienteils übergehen und dementsprechend lediglich Flächenabschnitte größerer, über den Übergangsbereich hinausreichender Folien sein.

Als Übergangsbereich 13 wird hier derjenige Bereich angesehen, der einerseits nicht mehr fest auf dem Spritzenkörper aufliegt bzw. nicht mehr klebend mit ihm verbunden ist, andererseits noch nicht an das massive Kunststoffformteil (beispielsweise ein Spritzgussteil) heranreicht. Somit ist der Übergangsbereich 13 ein biegsamer, noch nicht durch unterseitige Klebebeschichtungen an starren Formteilen fixierter Folienflächenabschnitt. Der Übergangsbereich 13 ermöglicht als Gelenkverbindung bzw. Schwenkverbindung das seitliche Auslenken, d.h. Aufrichten der (eigentlich tangential nach vorne vorstehenden) Nadelaufnahmeeinrichtung 15 gegenüber der ersten Richtung. Somit kann die Nadelaufnahmeeinrichtung 15 durch entsprechendes Biegen der Etikettenfolie bzw. Etikettenfolien im Übergangsbereich 13 nach oben oder unten geschwenkt werden. Insbesondere im Übergangsbereich kann die Etikettenfolie aus mehreren Schichten, d.h. Teilfolien bestehen.

Vorzugsweise umfasst die Etikettenfolie des Etiketts 10 eine erste Folie 1 und eine zweite Folie 2 (etwa aus Kunststoff oder Papier), wobei sich die Grundfläche der ersten Folie 1 vorzugsweise mindestens über den ersten Folienteil 11 und über den Übergangsbereich 13, vorzugsweise auch noch über die laterale Ausdehnung der Nadelaufnahmeeinrichtung 15 erstreckt. Die Grundfläche der zweiten Folie 2 umfasst vorzugsweise den Übergangsbereich 13 und die seitlichen Abmessungen der Nadelaufnahmeeinrichtung 15. Insbesondere im Übergangsbereich 13 sind vorzugsweise zwei Folien übereinander vorgesehen.

Bei den Ausführungsbeispielen gemäß den Figuren 4 bis 11 sind im Übergangsbereich 13 noch Einschnitte (in zumindest einer der Folien) dargestellt. Diese Einschnitte, die die Folien lokal unterbrechen oder zumindest (durch lokale Verminderung der Schichtdicke) schwächen, dienen dazu, später, wenn das Etikett um den zylindrischen Spritzenkörper geklebt ist, ein gezieltes Einstellen der Winkelposition der emporstehenden Nadelaufnahmeeinrichtung 15 zu bewirken. Bei dem Etikett wird durch das gezielte Anordnen einerseits von Schwächungselementen und andererseits von Verbindungsstegen bzw. Brückenbereichen an definierten Positionen innerhalb des Übergangsbereichs 13 ein definiertes Einstellverhalten der Nadelaufnahmeeinrichtung 15 hinsichtlich des Aufrichtwinkels erreicht, bei dem sie sich je nach anfänglicher Winkelposition weiter aufrichtet oder aber in eine von mehreren definierten Winkelpositionen zurückschnellt. Dieses Verhalten ist jedoch an der Anordnung, Dimensionierung, Größengestaltung und Formgebung der Schwächungselemente nicht direkt ablesbar, sondern das im Ergebnis erreichte Schwenkverhalten der Nadelaufnahmeeinrichtung 15 ergibt sich erst nach dem Aufspenden des Etiketts auf den zylindrischen, konischen oder in sonstiger Weise gekrümmten Außenmantel des Spritzenkörpers.

Gemäß der ersten Ausführungsform in Figur 4 ist ein erstes linienförmiges Schwächungselement 3 vorgesehen, und zwar eine bis fast zu beiden seitlichen Enden des Übergangsbereichs 13 durchlaufende, in sich gekrümmte Schwächungslinie.

Vorzugsweise ist in Figur 4 die erste Folie 1 eine obere Folie und die zweite Folie 2 eine zwischen ihr und dem Kunststoffformteil 14 angeordnete zweite Folie, vorzugsweise mit einer größeren Dicke als derjenigen der ersten Folie. Vorzugsweise wird durch das gekrümmte Schwächungselement lediglich die obere, erste Folie lokal durchtrennt. Es hat sich herausgestellt, dass eine Ausgestaltung der Schwächungselemente wie in Figur 4 dargestellt (und anhand der nachstehenden Figuren noch weitergebildet) sich eignet, um etwa das anhand Figur 2 erläuterte Einstellverhalten der Nadelaufnahmeeinrichtung 15 zu bewirken.

Es hat sich herausgestellt, dass die hier beschriebene Anordnung und Ausgestaltung gemäß Figur 4 und den darauffolgend beschriebenen Weiterbildungen besonders geeignet ist, um das anhand Figur 2 erläuterte Schwenkverhalten der Nadelaufnahmeeinrichtung 15 zu bewirken.

Figur 5 zeigt ein zweites Ausführungsbeispiel, bei dem zusätzlich zwei in axialer Richtung verlaufende zweite Schwächungselemente vorgesehen sind, die jeweils zum ersten Schwächungselement 3 beabstandet sind und in Richtung der Nadelaufnahmeeinrichtung 15, d.h. der ersten Richtung z weisen.

Sie sind jedoch auf entgegengesetzten Seiten des gekrümmten Schwächungselements 3 angeordnet. Diese drei Schwächungselemente sind vorzugsweise Durchschnitte einer oder mehrerer Folien des Etiketts im Übergangsbereich 13. Beispielsweise sind die kürzeren, axial verlaufenden Schwächungselemente 3 in beiden Folien 1, 2 ausgebildet.

Die beiden in axialer Richtung z verlaufenden Schwächungselemente sind als zweite Schwächungselemente 4 bzw. 4a und 4b bezeichnet. Wie anhand von Figur 5 erkennbar ist, sind beide zweiten Schwächungselemente 4a, 4b von dem ersten Schwächungselement 3 beabstandet. Somit existiert dazwischen jeweils ein erster Brückenbereich 7 auf beiden Seiten der Mitte des ersten Schwächungselements 3. Die ersten Brückenbereiche 7 dienen als Haltestege, um jeweils den rechten und den linken Folienabschnitt neben dem jeweiligen Schwächungselement 4a, 4b wieder zusammenzuführen, zumindest im Bereich unmittelbar an der Mitte des ersten Schwächungselements 3. Weiterhin erstreckt sich das erste Schwächungselement 3 nicht ganz bis zu den seitlichen Rändern des Übergangsbereichs 13, sondern stattdessen sind dort (ebenso wie in Figur 4) zweite Brückenbereiche 8 in derjenigen Folie, in der das erste Schwächungselement 3 ausgebildet ist, vorgesehen. Weiterhin ist zu erkennen, dass das erste Schwächungselement 3 ohne Unterbrechung durch die Mitte des Übergangsbereichs 13 durchläuft, wohingegen die beiden zweiten Schwächungselemente 4a, 4b in einem Abstand außerhalb des ersten Schwächungselements 3 enden.

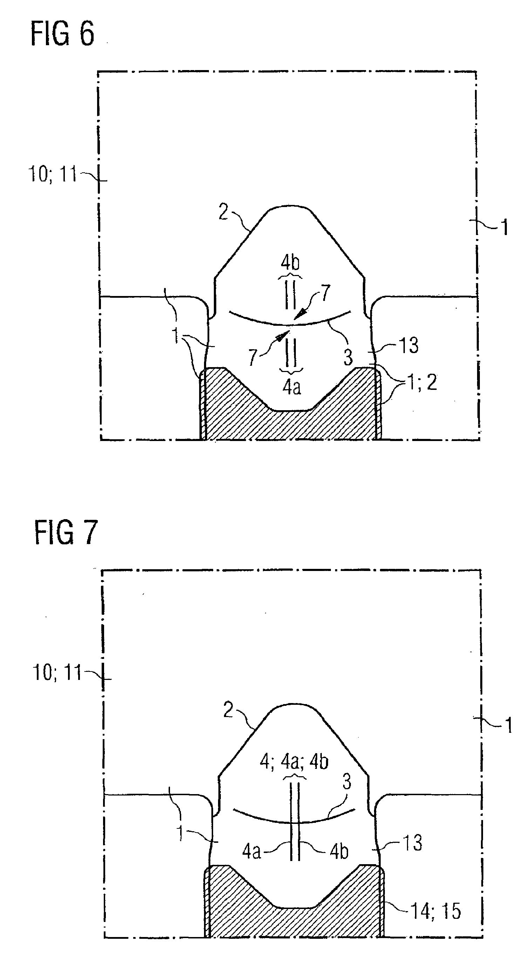

Figur 6 zeigt ein drittes Ausführungsbeispiel, bei dem anstelle jedes zweiten Schwächungselements 4a, 4b aus Figur 5 jeweils ein Paar von Schwächungselementen 4a, 4b vorgesehen ist. Somit besitzt der Übergangsbereich vier zweite Schwächungselemente, die jeweils paarweise parallel nebeneinander verlaufen und von dem ersten Schwächungselement 3 beabstandet sind.

Figur 7 zeigt ein viertes Ausführungsbeispiel, bei dem ebenfalls ein Paar zweiter Schwächungselemente 4a, 4b vorgesehen ist. Gegenüber Figur 6 jedoch kreuzt das Paar zweiter Schwächungselemente 4a, 4b das erste Schwächungselement 3, etwa als Überkreuzung in verschiedenen Folien und/oder als Durchschneidung in derselben Folie. Außerdem ist jedes der zweiten Schwächungselemente 4a, 4b so lang, dass es sich zwischen den äußeren Enden der jeweiligen zweiten Schwächungselemente 4a, 4b aus Figur 6 erstreckt. Auch in Figur 7 verlaufen die beiden zweiten Schwächungselemente 4a, 4b paarweise parallel nebeneinander.

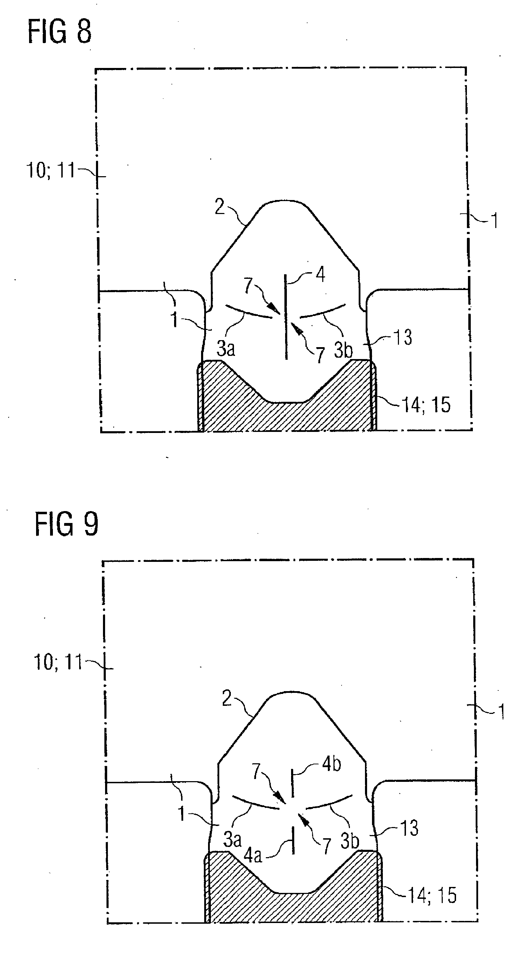

Figur 8 zeigt ein fünftes Ausführungsbeispiel, bei dem anstelle eines einzigen, fast bis an beide azimutalen Ränder des Übergangsbereichs 13 heranreichenden ersten Schwächungselements 3 zwei separate erste Schwächungselemente 3a, 3b (oder alternativ eine noch größere Anzahl erster Schwächungselemente) vorgesehen sind. Diese verlaufen beispielsweise quer oder ungefähr quer zur ersten Richtung z (oder alternativ geneigt, etwa diagonal dazu) und sind voneinander beabstandet. Zusätzlich kann optional noch zumindest ein zweites Schwächungselement 4 vorgesehen werden, das entlang der ersten Richtung z verläuft. Es kann in azimutaler Richtung zwischen beiden ersten Schwächungselementen 3a, 3b angeordnet sein. Seine axiale Position und Länge können so gewählt werden, dass es sich in axialer Richtung z teilweise auf beiden Seiten des Paares erster Schwächungselemente 3a, 3b erstreckt und somit auch durch die Mitte zwischen beiden ersten Schwächungselemente 3a, 3b hindurchführt.

Figur 9 zeigt ein sechstes Ausführungsbeispiel, bei dem gegenüber Figur 8 anstelle des einzigen axial verlaufenden zweiten Schwächungselements 4 zwei separate zweite Schwächungselemente 4a, 4b vorgesehen sind. Diese verlaufen entlang der ersten Richtung z auf jeweils einer Seite des Paares erster Schwächungselemente 3a, 3b und sind sowohl von diesen als auch voneinander beabstandet. Somit besitzt der Übergangsbereich vier Schwächungselemente 3a, 3b, 4a, 4b, die jeweils paarweise hintereinander verlaufen und von allen übrigen Schwächungselementen beabstandet sind.

Somit existiert in Figur 9 zwischen den Schwächungselementen 3a, 3b, 4a, 4b nur ein einziger, aber besonders stabiler erster Brückenbereich 7, wohingegen in den Figuren 5, 6 und 8 jeweils zwei separate erste Brückenbereiche 7 vorgesehen sind.

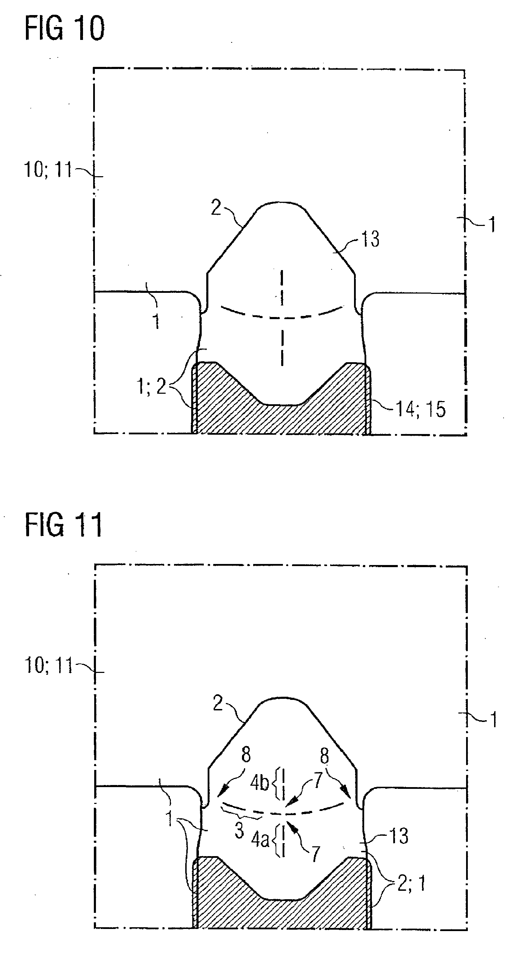

Figur 10 zeigt anhand des Ausführungsbeispiels aus Figur 5 eine alternative Ausführungsform, bei der die Schwächungselemente durch Perforationen gebildet sind. Auch die übrigen Ausführungsbeispiele der Figuren 4 und 6 bis 9 können auf die nachstehend beschriebene Weise abgewandelt werden. Die Schwächungselemente sind gemäß Figur 10 somit keine durchgehenden Schnitte, sondern bestehen aus einer Aneinanderreihung von Stanzlöchern, Spalten, Teilschnitten oder sonstigen Aussparungen oder Falzen entlang des Linienverlaufs der jeweiligen Schwächungslinie bzw. des jeweiligen Schwächungselements 3, 4a, 4b.

Figur 11 zeigt dieselbe Ausführungsform wie Figur 10, jedoch mit zusätzlichen Bezugszeichen zur Kennzeichnung der jeweiligen Schwächungselemente sowie der Brückenbereiche. Wie in Figur 11 erkennbar ist, ist jedes der Schwächungselemente 3, 4a, 4b in sich weiter unterteilt, d.h. nicht als durchgehende Schwächungs- oder Schnittlinie, sondern als Perforierung oder anderweitig unterbrochene Schwächungs- oder Schnittlinie ausgebildet. Die beiden zweiten Schwächungselemente 4a, 4b können beispielsweise in jeweils zwei oder mehr dicht aufeinander folgende Teillinien gegliedert sein. Dabei ist der Abstand zwischen diesen Teillinien innerhalb des jeweiligen zweiten Schwächungselements 4 kleiner als der Abstand dieses Schwächungselements zum ersten Schwächungselement 3, d.h. kleiner als die Breite des ersten Brückenbereichs 7 in axialer Richtung.

In Figur 11 ist ferner erkennbar, dass auch das erste Schwächungselement 3 als Perforierung aus (beispielsweise sechs) Teillinien ausgebildet ist. Auch hier ist der Abstand zwischen den Teillinien wesentlich kleiner als die Breite der zweiten Brückenbereiche 8 in Richtung quer zur axialen Haupterstreckungsrichtung der Nadelaufnahmeeinrichtung 15 und insbesondere auch kleiner als die axiale Ausdehnung der ersten Brückenbereiche 7. Dadurch entsteht eine Mikrostruktur der Schwächungselemente, obwohl die Schwächungselemente von außen betrachtet zunächst nur wie homogene, ununterbrochene Linien aussehen. Die Unterbrechungen zwischen den Einzellinien des ersten Schwächungselements sind insbesondere Sollbruchstellen bzw. Sollrissstellen, an denen die damit versehene Folie - für den Anwender nicht wahrnehmbar - durchreißt, sobald die Nadelaufnahmeeinrichtung des auf den Spritzenkörper aufgespendeten Etiketts erstmalig aufgerichtet wird. Es können wahlweise alle oder auch nur einige der Schwächungselemente, die in den Ausführungsbeispielen der Figuren 4 bis 9 vorgesehen sind, gemäß der Mikrostruktur aus Figur 10 bzw. 11 ausgebildet sein.

Je nachdem, in welche anfängliche Winkelstellung die Nadelaufnahmeeinrichtung 15 durch den Anwender von Hand gebracht wird, schnellt diese in eine von mehreren Winkelstellungen oder Winkelstellungsbereichen zurück, beispielsweise in die Winkelstellung A oder B aus Figur 2. Auch bei wiederholtem Hin- und Herbewegen des Nadelfängers von Hand garantiert die Gelenkverbindung im Übergangsbereich 13 ein zuverlässiges Schwenkverhalten, auch bei wiederholter Beanspruchung und Winkelverstellung durch den Anwender. Die Stabilität, Widerstandsfähigkeit und Haltbarkeit der Gelenkverbindung sind somit höher als bei herkömmlichen Spritzenetiketten.

Wie anhand dieser Ausführungsbeispiele verdeutlicht wird hier vorgeschlagen, in dem Übergangsbereich des Etiketts eine Anordnung mehrerer Schwächungselemente vorzusehen, wobei die Anordnung zumindest ein erstes Schwächungselement umfasst, durch welches mindestens eine Folie des Etiketts geschwächt, beispielsweise lokal unterbrochen wird, wobei außerdem das erste Schwächungselement in einer zur ersten Richtung abweichenden Orientierung angeordnet ist. Ein solches Schwächungselement ermöglicht bei einem um einen Spritzenkörper gewickelten Etikett ein definiertes Aufrichten und Einstellen definierter Winkelpositionen, die die Handhabung der Spritze erleichtern. Gemäß einer Ausführungsform können zwei separate erste Schwächungselemente vorgesehen sein, insbesondere in zueinander spiegelbildlicher Anordnung.

Zweite Schwächungselemente können auf zueinander entgegengesetzten Seiten des ersten Schwächungselements oder eines Paares erster Schwächungselemente angeordnet und jeweils von dem oder den ersten Schwächungselementen sowie voneinander beabstandet sein. Dabei kann insbesondere eines der beiden zweiten Schwächungselemente in Richtung der Spritzennadel vor dem ersten Schwächungselement, das andere zweite Schwächungselement in Richtung der Spritzennadel hingegen hinter dem ersten Schwächungselement angeordnet sein.

Die Kombinationen aus Anzahl, Formgebung, gegenseitiger Relativposition sowie Trennung der Schwächungselemente ermöglichen es, ein unter Verwendung von Folien hergestelltes Etikett im Übergangsbereich dergestalt auszubilden, dass die Winkelstellung oder Winkelstellungen, zu denen der schwenkbare Nadelschutz tendieren soll, sich auch nach wiederholtem Hin- und Herbewegen zuverlässig wieder einstellt, und zwar ohne dass eine der Folien im Übergangsbereich des Etiketts außerhalb von Sollbruchstellen bricht oder ermüdet und ohne dass die Spannung und Federkraft der Folien mit der Zeit verloren geht. Somit ermöglicht es die oben beschriebene Anordnung von Schwächungselementen, mit Hilfe von Folien eine dauerhafte und zuverlässige Federkraft im Übergangsbereich in Richtung der vordefinierten Winkelstellungen zu erzielen.

Die ersten Schwächungselement und/oder zweiten Schwächungselemente können als Schnitte, Perforationen oder sonstige Aussparungen, die mindestens eine Folie des Etiketts im Übergangsbereich lokal unterbrechen oder schwächen, ausgebildet sind. Im Bereich der Schnittlinien wird die jeweilige Folie vollständig durchtrennt oder nur teilweise eingeschnitten. Die ersten Schwächungselement und/oder zweiten Schwächungselemente können auch als Perforierung ausgebildet sein; in diesem Fall besteht jedes dieser Schwächungselemente aus einer Folge mehrerer, mindestens zweier Teilschnitte, Löcher, Ausstanzungen oder sonstiger lokal abgegrenzter Ausnehmungen oder Materialtrennungen. Dabei sind die jeweiligen Ausnehmungen oder sonstigen Aussparungen eines betreffenden Schwächungselements jedoch so nah aneinander angeordnet, dass ihr Abstand wesentlich kleiner ist als der Abstand des gesamten Schwächungselements zu den übrigen. Insbesondere sind vorzugsweise die einzelnen Schnitte oder Materialaussparungen desselben Schwächungselements so dicht nebeneinander angeordnet, dass ihr Abstand mit bloßem Auge nicht mehr erkennbar ist und die innere Struktur des Schwächungselements somit bei der bloßen Betrachtung verborgen bleibt.

Zwischen den jeweiligen Schwächungselementen können Brückenbereiche vorgesehen sein, die trotz der Schwächungselemente zu einer gewissen Stabilisierung führen, die mit dem gewünschten Ausrichtverhalten in Bezug auf die Winkeleinstellungen der Nadelaufnahmeeinrichtung verträglich ist. Die Brückenbereiche tragen zur Spannung innerhalb des Übergangsbereichs des Etiketts bei Einstellung eines ungünstigen Schwenkwinkels bei und fördern dadurch ein Vor- oder Zurückschnappen in die eine oder andere, näher oder weiter von der Spritzennadel entfernte Winkelstellung der Nadelaufnahmeeinrichtung.

Das Etikett kann eine erste Folie und eine zweite Folie aufweisen, wobei die Grundfläche beider Folien mindestens den Übergangsbereich mit umfasst. Somit kann die gewünschte Orientierung der Nadelaufnahmeeinrichtung in Bezug auf ein Umklappen, Zurückschnappen in gewünschte Winkelstellungen optimiert werden, indem im Übergangsbereich beide Folien in unterschiedlicher Weise mit den Schwächungselementen versehen werden. Die Folien können etwa Kunststofffolien oder Papierfolien sein. Das zumindest eine erste Schwächungselement kann in der zweiten Folie, statt in der ersten Folie ausgebildet sein. Diese zweite Folie kann beispielsweise eine untere, vorzugsweise auch dickere oder verbindungssteifere Folie sein. Weiterhin kann das zumindest eine zweite Schwächungselement in beiden Folien zugleich ausgebildet sein. Dadurch vergrößert sich der Einfluss des zweiten Schwächungselements auf das Winkelausrichtverhalten.

Im Übrigen kann das Kunststoffformteil ein Spritzgussteil sein und Widerhaken auf der Unterseite zur Aufnahme der Spritzennadel aufweisen.

Es kann vorgesehen sein, dass sich die Grundfläche der ersten Folie über den ersten Folienteil, den Übergangsbereich und die Nadelaufnahmeeinrichtung erstreckt. Insbesondere kann der erste Folienteil des Etiketts ausschließlich aus der ersten, vorzugsweise oberen bzw. äußeren Folie gebildet sein. Der erste Folienteil des Etiketts ist derjenige Flächenbereich mit der größten Grundfläche, da er um den Umfang des Spritzenkörpers umgewickelt wird. Der Übergangsbereich und/oder der Folienabschnitt im Bereich über dem Kunststoffformkörper hingegen werden sich nur über einen gewissen, kleineren azimutalen Umfangswinkel um die Spritzennadel erstrecken. Weiterhin kann vorgesehen sein, dass sich die Grundfläche der zweiten Folie über den Übergangsbereich und die Nadelaufnahmeeinrichtung erstreckt. Beispielsweise kann sich die zweite Folie über die gesamte Außenseite des Kunststoffformkörpers, die (bis auf eventuelle Aussparungen oder Vertiefungen) überwiegend glatt ausgebildet ist, erstrecken. Weiterhin kann sich die zweite, vorzugsweise untere Folie über den Übergangsbereich hinaus bis zu einem unmittelbar anschließenden, in erster Näherung halbrunden, kegelförmigen oder in sonstiger Weise auslaufenden Teilbereich des ersten Folienbereichs erstrecken.

Die zweite Folie kann eine Schichtdicke von zwischen 50 und 200 µm besitzen, wohingegen die erste, vorzugsweise äußere Folie mit einer Schichtdicke von beispielsweise 25 bis 100 µm ausgebildet sein kann.

Vorzugsweise ist vorgesehen, dass die Nadelaufnahmeeinrichtung für die Spritzennadel mit Hilfe des Übergangsbereichs, durch den sie mit dem ersten Folienbereich verbunden ist, zwischen einer ersten Winkelstellung und einer zweiten Winkelstellung, in der die Nadelaufnahmeeinrichtung jeweils einschnappt, verschwenkbar ist. Zwischen beiden Winkelstellungen bzw. Winkelstellungsbereichen liegt dann ein Bereich weniger stabiler Winkelstellungen, von dem aus ein Vor- oder Zurückschnappen in die bevorzugte vordere oder hintere Winkelstellung automatisch erfolgt. Dies erleichtert den Gebrauch der Spritzen während bzw. nach der Injektion. Vorzugsweise ist vorgesehen, dass die erste Winkelstellung im Bereich zwischen 0° und 20° relativ zur ersten Richtung liegt und dass die zweite Winkelstellung im Bereich zwischen 50 und 80° relativ zur ersten Winkelstellung liegt.

Ein mit dem vorgeschlagenen Etikett bestückter, beispielsweise beklebter Spritzenkörper erleichtert die Injektion und die Spritzenhandhabung, da aufgrund der Ausbildung des Übergangsbereichs die Einstellung der Nadelaufnahmeeinrichtung, die ja erst nach dem Injizieren benötigt wird, in eine vordefinierte Winkelstellung (insbesondere in Richtung schräg geneigt zur Richtung der Spritzennadel) automatisch von selbst erfolgt, und zwar selbst nach mehrmaligem Hin- und Herschwenken. Dadurch ist die in vordefinierter Winkelstellung abstehende Nadelaufnahmeeinrichtung beim Ansetzen der Spritze und Injizieren, auch in größerer Tiefe unter der Haut, nicht im Wege.

### Bezugszeichenliste

- 1: erste Folie
- 2: zweite Folie
- 3; 3a, 3b: erstes Schwächungselement
- 4; 4a, 4b: zweites Schwächungselement
- 7: erster Brückenbereich
- 8: zweiter Brückenbereich
- 10: Etikett
- 11: erster Folienteil
- 12: zweiter Folienteil
- 13: Übergangsbereich
- 14: Kunststoffformteil
- 15: Nadelaufnahmeeinrichtung
- 19: Spritzennadel
- 20: Spritzenkörper
- A: erste Winkelstellung
- B: zweite Winkelstellung
- z: erste Richtung

## Patentansprüche

1. Etikett (10) zum Anbringen an einem Spritzenkörper (20), wobei das Etikett (10) Folgendes aufweist:
- einen ersten Folienteil (11), der um einen Spritzenkörper (20) umwickelbar ist,
- eine Nadelaufnahmeeinrichtung (15) für eine Spritzennadel (19) zum Schutz vor Verletzungen durch die Spritzennadel (19), wobei die Nadelaufnahmeeinrichtung (15) zumindest einen zweiten Folienteil (12) und ein Kunststoffformteil (14), das gegen die Spritzennadel (19) seitlich andrückbar ist, aufweist, und
- einen Übergangsbereich (13), durch den die Nadelaufnahmeeinrichtung (15) schwenkbar mit dem ersten Folienteil (11) verbunden ist, wobei der Übergangsbereich (13) in einer ersten Richtung (z) seitlich über den ersten Folienteil (11) hinausragt und wobei das Etikett (10) in dem Übergangsbereich (13) eine oder mehrere Folien (1, 2) aufweist,
**dadurch gekennzeichnet, dass**
- im Übergangsbereich (13) des Etiketts (10) in mindestens einer Folie (1; 2) zumindest ein erstes Schwächungselement (3) vorgesehen ist,
- wobei das zumindest eine erste Schwächungselement (3) ein Schwächungselement ist, welches eine von der ersten Richtung (z) abweichende Orientierung besitzt und ein definiertes Aufrichten und Einstellen der Nadelaufnahmeeinrichtung (15) in einer oder mehreren definierten Winkelpositionen gegenüber der ersten Richtung (z) ermöglicht.

2. Etikett nach Anspruch 1,
**dadurch gekennzeichnet, dass**
der Übergangsbereich (13) des Etiketts (10) in mindestens einer Folie (1; 2) zumindest zwei erste Schwächungselemente (3a; 3b) aufweist, die eine von der ersten Richtung (z) abweichende Orientierung besitzen.

3. Etikett nach Anspruch 1 oder 2,
**dadurch gekennzeichnet, dass**
das erste Schwächungselement (3) oder die ersten Schwächungselemente (3a, 3b) insgesamt quer zur ersten Richtung (z) verlaufen.

4. Etikett nach einem der Ansprüche 1 bis 3,
**dadurch gekennzeichnet, dass**
das erste Schwächungselement (3) oder die ersten Schwächungs-elemente (3a, 3b) einen gekrümmten Verlauf besitzen, wobei ein in der Mitte des Übergangsbereichs angeordneter Teil des oder der ersten Schwächungselemente (3; 3a, 3b) näher an die Nadelaufnahmeeinrichtung (15) für die Spritzennadel (19) heranreicht als zwei äußere Enden des oder der ersten Schwächungselemente (3; 3a, 3b).

5. Etikett nach einem der Ansprüche 1 bis 4,
**dadurch gekennzeichnet, dass**
im Übergangsbereich (13) des Etiketts (10) in mindestens einer Folie (1; 2) mindestens ein zweites Schwächungselement (4; 4a, 4b) vorgesehen ist, wobei das zweite Schwächungselement (4) oder die zweiten Schwächungselemente (4a, 4b) entlang der ersten Richtung (z) verlaufen.

6. Etikett nach Anspruch 5,
**dadurch gekennzeichnet, dass**
dass mehrere zweite Schwächungselemente (4; 4a, 4b) auf zueinander entgegengesetzten Seiten des zumindest einen ersten Schwächungselements (3; 3a, 3b) angeordnet und jeweils von dem zumindest einen zumindest einen ersten Schwächungselement (3; 3a, 3b) beabstandet sind.

7. Etikett nach Anspruch 6,
**dadurch gekennzeichnet, dass**
dass die zweiten Schwächungselemente (4a, 4b) nebeneinander angeordnet sind und jeweils das zumindest eine erste Schwächungselement (3) kreuzen oder schneiden.

8. Etikett nach Anspruch 6,
**dadurch gekennzeichnet, dass**
dass auf zueinander entgegengesetzten Seiten des zumindest einen ersten Schwächungselements (3; 3a, 3b) jeweils zwei zweite Schwächungselemente (4a, 4b) angeordnet sind, die jeweils von dem zumindest einen ersten Schwächungselement (3; 3a, 3b) beabstandet sind.

9. Etikett nach einem der Ansprüche 1 bis 8,
**dadurch gekennzeichnet, dass**
das die ersten und/oder zweiten Schwächungselemente (3; 3a, 3b; 4; 4a, 4b) als Schwächungslinien, und zwar vorzugsweise als Schnittlinien, Perforierungen oder sonstige Aussparungen ausgebildet sind, die mindestens eine Folie (1, 2) des Etiketts (10) im Übergangsbereich (13) lokal unterbrechen.

10. Etikett nach einem der Ansprüche 5 bis 9,
**dadurch gekennzeichnet, dass**
im Übergangsbereich (13) zumindest eine Folie (1, 2) zwischen dem zumindest einen ersten Schwächungselement (3; 3a, 3b) und dem zumindest einen zweiten Schwächungselement (4; 4a, 4b) mindestens einen ersten Brückenbereich (7) unverminderter Schichtdicke aufweist.

11. Etikett nach einem der Ansprüche 5 bis 10,
**dadurch gekennzeichnet, dass**
das Etikett (10) zumindest im Übergangsbereich (13) eine erste, obere Folie (1) und eine zwischen der ersten Folie (1) und dem Kunststoffformteil (14) angeordnete, vorzugsweise dickere und/oder verwindungssteifere zweite Folie (2) aufweist,
- wobei das zumindest eine erste Schwächungselement (3; 3a, 3b) in der zweiten Folie (2), nicht aber in der ersten Folie (1) ausgebildet ist und
- wobei das zumindest eine zweite Schwächungselement (4; 4a, 4b) sowohl in der ersten Folie (1) als auch in der zweiten Folie (2) ausgebildet ist.

12. Etikett nach Anspruch 11,
**dadurch gekennzeichnet, dass**
sich die Grundfläche der ersten Folie (1) über den ersten Folienteil (11), den Übergangsbereich (13) und die Nadelaufnahmeeinrichtung (15) erstreckt und dass sich die Grundfläche der zweiten Folie (2) über den Übergangsbereich (13) und die Nadelaufnahmeeinrichtung (15) erstreckt.

13. Etikett nach einem der Ansprüche 1 bis 12,
**dadurch gekennzeichnet, dass**
die Nadelaufnahmeeinrichtung (15) für die Spritzennadel (19) mit Hilfe des Übergangsbereichs (13), durch den sie mit dem ersten Folienteil (11) verbunden ist, zwischen einer ersten Winkelstellung (A) und einer zweiten Winkelstellung (B), in der die Nadelaufnahmeeinrichtung (15) jeweils stehen bleibt, verschwenkbar ist.

14. Etikett nach Anspruch 13,
**dadurch gekennzeichnet, dass**
die erste Winkelstellung (A) im Bereich zwischen 0° und 20° relativ zur ersten Richtung (z) liegt und dass die zweite Winkelstellung (B) im Bereich zwischen 50° und 80° relativ zur ersten Richtung (z) liegt.

15. Spritzenkörper (20) mit einer Spritzennadel,
**dadurch gekennzeichnet, dass**
der Spritzenkörper (20) mit einem Etikett (10) nach einem der Ansprüche 1 bis 14 versehen ist, wobei der erste Folienteil (11) des Etiketts (10) entlang eines Umfangs des Spritzenkörpers (20) aufgeklebt ist.

## Claims

1. A label (10) for attachment to a syringe body (20), the label (10) comprising:
- a first foil section (11) which can be wrapped around a syringe body (20),
- a needle receiving device (15) for a syringe needle (19) to provide protection against injury by the syringe needle (19), the needle receiving device (15) comprising at least one second foil section (12) and a molded plastic part (14), the latter being able to be pressed laterally against the syringe needle (19), and
- a transition zone (13) through which the needle receiving device (15) is connected to the first foil section (11) in a pivotable manner, the transition zone (13) projecting laterally beyond the first foil section (11) in a first direction (z), and the label (10) comprising one or more foils (1, 2) in the transition zone (13),
**characterized in that**
- at least one first weakening element (3) is provided in the transition zone (13) of the label (10) in at least one foil (1; 2),
- the at least one first weakening element (3) being a weakening element which has an orientation deviating from the first direction (z) and allows a defined deployment and positioning of the needle receiving device (15) in one or more defined angular positions with respect to the first direction (z).

2. The label according to claim 1,
**characterized in that**
the transition zone (13) of the label (10) comprises at least two first weakening elements (3a; 3b) in at least one foil (1; 2), which have an orientation deviating from the first direction (z).

3. The label according to claim 1 or 2,
**characterized in that**
the first weakening element (3) or the first weakening elements (3a, 3b) as a whole extend(s) transverse to the first direction (z).

4. The label according to any of the claims 1 to 3,
**characterized in that**
the first weakening element (3) or the first weakening elements (3a, 3b) has/have a curved line profile, a part of the first weakening element(s) (3; 3a, 3b), which is arranged in the middle of the transition zone, coming closer to the needle receiving device (15) for the syringe needle (19) than two outer ends of the first weakening element(s) (3; 3a, 3b).

5. The label according to any of the claims 1 to 4,
**characterized in that**
at least one second weakening element (4) is provided in the transition zone (13) of the label (10) in at least one foil (1; 2), the second weakening element (4) or the second weakening elements (4a, 4b) extending along the first direction (z).

6. The label according to claim 5,
**characterized in that**
several second weakening elements (4; 4a, 4b) are arranged on opposite sides of the at least one first weakening element (3; 3a, 3b) and are spaced from the at least one first weakening element (3; 3a, 3b) in each case.

7. The label according to claim 6,
**characterized in that**
the second weakening elements (4a, 4b) are arranged side by side and cross or intersect the at least one first weakening element (3) in each case.

8. The label according to claim 6,
**characterized in that**
on opposite sides of the at least one first weakening element (3; 3a, 3b), two second weakening elements (4a, 4b) are arranged in each case, each being spaced from the at least one first weakening element (3; 3a, 3b).

9. The label according to any of the claims 1 to 8,
**characterized in that**
the first and/or second weakening elements (3; 3a, 3b; 4; 4a, 4b) are formed as weakening lines, preferably as cutting lines, perforations or any other recesses, creating a local interruption in the at least one foil (1, 2) of the label (10) in the transition zone (13).

10. The label according to any of the claims 5 to 9,
**characterized in that**,
in the transition zone (13), at least one foil (1, 2) between the at least one first weakening element (3; 3a, 3b) and the at least one second weakening element (4; 4a, 4b) comprises at least one first bridge area (7) having an undiminished layer thickness.

11. The label according to any of the claims 5 to 10,
**characterized in that**
the label (10), at least in the transition zone (13), comprises a first, upper foil (1) and a second foil (2) which is arranged between the first foil (1) and the molded plastic part (14) and preferably has a higher thickness and/or a higher torsional rigidity,
- the at least one first weakening element (3; 3a; 3b) is formed in the second foil (2), but not in the first foil (1) and
- the at least one second weakening element (4; 4a; 4b) is formed in the first foil (1) as well as in the second foil (2).

12. The label according to claim 11,
**characterized in that**
the base area of the first foil (1) extends over the first foil section (11), the transition zone (13) and the needle receiving device (15) and the base area of the second foil (2) extends over the transition zone (13) and the needle receiving device (15).

13. The label according to any of the claims 1 to 12,
**characterized in that**
the needle receiving device (15) for the syringe needle (19) can be pivoted by means of the transition zone (13), by which it is connected to the first foil section (11), between a first angular position (A) and a second angular position (B) in which the needle receiving device (15) comes to a stop in each case.

14. The label according to claim 13,
**characterized in that**
the first angular position (A) is in the range between 0° and 20° relative to the first direction (z) and the second angular position (B) is in the range between 50° and 80° relative to the first direction (z).

15. A syringe body (20) comprising a syringe needle,
**characterized in that**
the syringe body (20) is provided with a label (10) according to any of the claims 1 to 14, the first foil section (11) of the label (10) being affixed along a circumference of the syringe body (20).

## Revendications

1. Étiquette (10) destinée à être appliquée sur un corps de seringue (20), l'étiquette (10) présentant ce qui suit :
- une première partie en film (11) qui est apte à être enroulée autour d'un corps de seringue (20),
- un dispositif de logement d'aiguille (15) pour une aiguille de seringue (19) pour se protéger de blessures causées par l'aiguille de seringue (19), le dispositif de logement d'aiguille (15) présentant au moins une deuxième partie en film (12) et une partie moulée en matière plastiquée (14) apte à être pressée latéralement contre l'aiguille de seringue (19), et
- une zone de transition (13) par laquelle le dispositif de logement d'aiguille (15) est relié de manière pivotante à la première partie en film (11), la zone de transition (13) dépassant latéralement de la première partie en film (11) dans une première direction (z) et l'étiquette (10) présentant un ou plusieurs films (1, 2) dans la zone de transition,
**caractérisée en ce que**
- au moins un premier élément d'affaiblissement (3) est prévu dans la zone de transition (13) de l'étiquette (10) dans au moins un film (1 ; 2),
- l'au moins un premier élément d'affaiblissement (3) étant un élément d'affaiblissement qui possède une orientation divergente de la première direction (z) et permet de dresser ou de régler de manière définie le dispositif de logement d'aiguille (15) dans une ou plusieurs positions angulaires définies par rapport à la première direction (z).

2. Étiquette selon la revendication 1,
**caractérisée en ce que**
la zone de transition (13) de l'étiquette (10) présente, dans au moins un film (1 ; 2), au moins deux premiers éléments d'affaiblissement (3a ; 3b) qui possèdent une orientation divergente de la première direction (z).

3. Étiquette selon la revendication 1 ou 2,
**caractérisée en ce que**
le premier élément d'affaiblissement (3) ou les premiers éléments d'affaiblissement (3a, 3b) décrivent un tracé sensiblement perpendiculaire à la première direction (z) .

4. Étiquette selon l'une des revendications 1 à 3,
**caractérisée en ce que**
le premier élément d'affaiblissement (3) ou les premiers éléments d'affaiblissement (3a, 3b) décrivent un tracé courbe, une partie du ou des premiers éléments d'affaiblissement (3 ; 3a, 3b) disposée au milieu de la zone de transition arrivant plus près du dispositif de logement d'aiguille (15) pour l'aiguille de seringue (19) que deux extrémités extérieures du ou des premiers éléments d'affaiblissement (3 ; 3a, 3b).

5. Étiquette selon l'une des revendications 1 à 4,
**caractérisée en ce que**
au moins un deuxième élément d'affaiblissement (4 ; 4a, 4b) est prévu dans la zone de transition (13) de l'étiquette (10) dans au moins un film (1 ; 2), le deuxième élément d'affaiblissement (4) ou les deuxièmes éléments d'affaiblissement (4a, 4b) décrivant un tracé le long de la première direction (z).

6. Étiquette selon la revendication 5,
**caractérisée en ce que**
plusieurs deuxièmes éléments d'affaiblissement (4 ; 4a, 4b) sont disposés sur des côtés opposés l'un à l'autre de l'au moins un premier élément d'affaiblissement (3 ; 3a, 3b) et sont distants respectivement de l'au moins un premier élément d'affaiblissement (3 ; 3a, 3b).

7. Étiquette selon la revendication 6,
**caractérisée en ce que**
les deuxièmes éléments d'affaiblissement (4a, 4b) sont disposés l'un à côté de l'autre et croisent ou coupent respectivement l'au moins un premier élément d'affaiblissement (3).

8. Étiquette selon la revendication 6,
**caractérisée en ce que**
respectivement deux deuxièmes éléments d'affaiblissement (4a, 4b) qui sont distants respectivement de l'au moins un premier élément d'affaiblissement (3 ; 3a, 3b) sont disposés sur des côtés opposés l'un à l'autre de l'au moins un premier élément d'affaiblissement (3 ; 3a, 3b).

9. Étiquette selon l'une des revendications 1 à 8,
**caractérisée en ce que**
les premiers et/ou deuxièmes éléments d'affaiblissement (3 ; 3a, 3b ; 4 ; 4a, 4b) sont constitués comme lignes d'affaiblissement, à savoir de préférence comme lignes d'intersection, perforations ou autres évidements, qui interrompent localement au moins un film (1, 2) de l'étiquette (10) dans la zone de transition (13).

10. Étiquette selon l'une des revendications 5 à 9,
**caractérisée en ce que**
dans la zone de transition (13), au moins un film (1, 2) entre l'au moins un premier élément d'affaiblissement (3 ; 3a, 3b) et l'au moins un deuxième élément d'affaiblissement (4 ; 4a, 4b) présente au moins une première zone de pont (7) d'épaisseur de couche non diminuée.

11. Étiquette selon l'une des revendications 5 à 10,
**caractérisée en ce que**
l'étiquette (10) présente, au moins dans la zone de transition (13), un premier film supérieur (1) et un deuxième film (2) disposé entre le premier film (1) et la partie moulée en matière plastique, de préférence plus épais et/ou plus résistant à la torsion,
- l'au moins un premier élément d'affaiblissement (3 ; 3a, 3b) étant constitué dans le deuxième film (2), mais pas dans le premier film (1) et
- l'au moins un deuxième élément d'affaiblissement (4 ; 4a, 4b) étant constitué aussi bien dans le premier film (1) que dans le deuxième film (2).

12. Étiquette selon la revendication 11,
**caractérisée en ce que**
la surface de base du premier film (1) s'étend sur la première partie en film (11), la zone de transition (13) et le dispositif de logement d'aiguille (15) et **en ce que** la surface de base du deuxième film (2) s'étend sur la zone de transition (13) et le dispositif de logement d'aiguille (15).

13. Étiquette selon l'une des revendications 1 à 12,
**caractérisée en ce que**
le dispositif de logement d'aiguille (15) pour l'aiguille de seringue (19) peut, à l'aide de la zone de transition (13) par laquelle il est relié à la première partie en film (11), être basculé entre une première position angulaire (A) et une deuxième position angulaire (B) dans laquelle le dispositif de logement d'aiguille (15) reste positionné respectivement.

14. Étiquette selon la revendications 13,
**caractérisée en ce que**
la première position angulaire (A) se situe dans la plage comprise entre 0° et 20° par rapport à la première direction (z) et **en ce que** la deuxième position angulaire (B) se situe dans la plage comprise entre 50° et 80° par rapport à la première direction (z).

15. Corps de seringue (20) avec une aiguille de seringue,
**caractérisé en ce que**
le corps de seringue (20) est pourvu d'une étiquette (10) selon l'une des revendications 1 à 14, la première partie en film (11) de l'étiquette (10) étant collée le long d'une circonférence du corps de seringue (20).
